Europäisches Patentamt

European Patent Office

Office européen des brevets

(11) Numéro de publication: **0 081 196**
**B1**

(12) **FASCICULE DE BREVET EUROPEEN**

(19)

(45) Date de publication du fascicule du brevet:
02.05.85

(21) Numéro de dépôt: 82111128.3

(22) Date de dépôt: 02.12.82

(51) Int. Cl.⁴: **C 12 M 1/40, C 13 K 1/06**

(54) Dispositif pour obtenir du sucre fermentescible à partir de déchets organiques.

(30) Priorité: 09.12.81 FR 8123012

(43) Date de publication de la demande:
15.06.83 Bulletin 83/24

(45) Mention de la délivrance du brevet:
02.05.85 Bulletin 85/18

(84) Etats contractants désignés:
BE CH DE FR GB IT LI NL SE

(56) Documents cités:
FR - A - 1 589 364
FR - A - 2 218 344
US - A - 4 226 937

(73) Titulaire: **COMPAGNIE GENERALE D'ELECTRICITE**
**Société anonyme dite:, 54, rue La Boétie, F-75382 Paris**
**Cedex 08 (FR)**

(72) Inventeur: **Lerner, Pierre, 58, Allée du Fief Lambert**
**Chevry, F-91190 Gif-Sur-Yvette (FR)**
Inventeur: **Petitbon, Alain, 2, avenue de l'Essonne,**
**F-91130 Ris-Orangis (FR)**

(74) Mandataire: **Weinmiller, Jürgen et al, Zeppelinstrasse 63,**
**D-8000 München 80 (DE)**

## Description

La présente invention concerne un dispositif pour obtenir du sucre fermentescible à partir de déchets organiques, comportant des moyens pour mettre en contact un liquide contenant en suspension des déchets organiques avec une première enzyme apte à transformer ces déchets en un mélange de sucres, ce mélange contenant un sucre fermentescible, et des moyens pour mettre en contact le mélange avec une deuxième enzyme immobilisée dans une céramique poreuse, de façon à enrichir le mélange en sucre fermentescible.

Un dispositif de ce type comporte une cuve contenant une solution aqueuse de la première enzyme, la température et l'acidité de cette solution étant réglées à des valeurs prédéterminées. On ajoute ensuite dans la solution une poudre de déchets organiques, agricoles ou végétaux, pour former une suspension. On obtient ainsi, par hydrolyse, un sirop contenant des sucres dont l'un est fermentescible. Le dispositif comporte en outre un lit fixe formé d'une pluralité de pièces de céramiques poreuses dans lesquelles est immobilisée la deuxième enzyme. On règle alors la température et l'acidité du sirop à d'autres valeurs prédéterminées, puis on fait passer le sirop à travers le lit de façon à enrichir ce sirop en sucre fermentescible. Celui-ci est destiné à être transformé par fermentation en alcool ou en méthane.

Le dispositif décrit ci-dessus présente des inconvénients. En effet, ce système est relativement complexe. De plus, la première enzyme est souvent maintenue dans le sirop et se trouve donc perdue. Si l'on veut récupérer cette première enzyme, il est nécessaire de procéder, avant passage à travers le lit fixe, à une ultrafiltration du sirop, ce qui constitue une opération coûteuse. Pour régénérer les pièces poreuses du lit fixe après épuisement de la deuxième enzyme, il est nécessaire de défaire ce lit, puis de le refaire, ces deux opérations étant relativement longues. Le débit de passage du sirop à travers le lit est faible. Enfin, la traversée du lit par le sirop peut s'effectuer à travers des canaux préférentiels, ce qui nuit au rendement.

La présente invention a pour but de pallier ces inconvénients.

La présente invention a pour objet un dispositif pour obtenir du sucre fermentescible à partir de déchets organiques, comportant:
- des moyens pour mettre en contact un liquide contenant en suspension des déchets organiques avec une première enzyme apte à transformer ces déchets en un mélange de sucres, ce mélange contenant un sucre fermentescible, et
- des moyens pour mettre en contact le mélange avec une deuxième enzyme immobilisée dans une céramique poreuse, de façon à enrichir le mélange en sucre fermentescible,
caractérisé en ce qu'il comporte:
- une cuve contenant un bain dudit liquide dans lequel est immergée une céramique microporeuse, la deuxième enzyme étant immobilisée dans cette céramique,
- des granulés microporeux de céramique en suspension dans le bain, la première enzyme étant immobilisée dans ces granulés,
- un tube dont la paroi poreuse comporte un support tubulaire en céramique macroporeuse, une cloison étanche pour obturer hermétiquement une extrémité du tube, l'autre extrémité du tube étant reliée à une canalisation de recyclage munie d'une pompe et aboutissant au-dessus du bain, le tube étant immergé dans le bain et la pompe étant capable d'aspirer dans la canalisation la partie liquide du bain à travers la paroi du tube et de renvoyer le liquide aspiré dans le bain,
- des moyens pour verser dans le bain le liquide contenant les déchets organiques en suspension, les pores du support du tube étant capables de retenir, pendant l'aspiration, les granulés et les déchets organiques subsistant dans le bain,
- des moyens pour maintenir la température et l'acidité du bain contenant les granulés et les déchets organiques à des valeurs prédéterminées qui permettent, d'une part, la transformation des déchets organiques en un mélange de sucres sous l'action de la première enzyme et, d'autre part, l'enrichissement du mélange en sucre fermentescible sous l'action de la deuxième enzyme, et
- des moyens commandables pour prélever le sucre fermentescible à la sortie du tube.

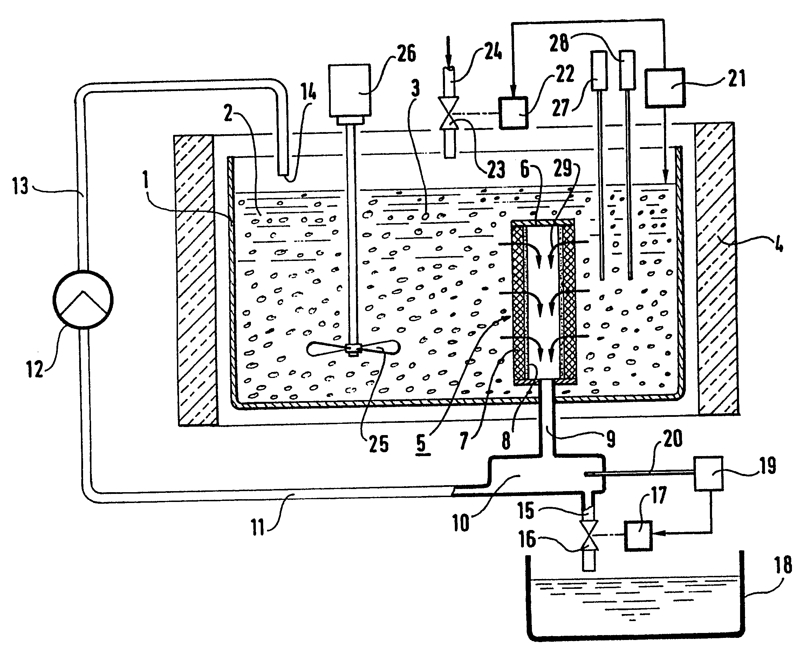

Des formes particulières d'exécution de l'objet de la présente invention sont décrites ci-dessous, à titre d'exemple, en référence au dessin annexé dans lequel la figure unique représente schématiquement un mode de réalisation du dispositif selon l'invention.

Sur la figure est représentée une cuve 1 contenant un bain 2 formé d'une suspension aqueuse de granulés microporeux 3 en céramique, par exemple en alumine γ. La grosseur des granulés peut être de l'ordre de quelques dizaines de microns. Dans ces granulés est immobilisée une première enzyme apte à transformer les produits organiques en sucres. La cuve 1 est placée dans le volume interne d'un four vertical 4 muni de moyens d'alimentation électriques non représentés.

Dans le bain 2 est plongé un tube en céramique 5. Une extrémité du tube 5 est fermée par une cloison étanche 6. Le tube proprement dit comporte un support tubulaire macroporeux 7 qui peut être en alumine α et dont les pores ont une dimension de quelques microns. Sur la surface cylindrique interne de ce support est fixée une membrane tubulaire microporeuse 8, par exemple en alumine γ. Les pores de cette membrane ont une dimension inférieure à 0,2 μm. A l'intérieur des pores de cette membrane est immobilisée une deuxième enzyme. A l'autre extrémité du tube est fixée une canalisation 9 traversant la paroi de la cuve 1 et mettant en communication le volume interne du tube 5 avec un vase d'expansion 10. Le vase 10 comporte une ouverture reliée par une canalisation 11 à l'entrée d'une pompe 12. A la sortie de la pompe 12 est branchée une canalisation 13 dont l'extrémité libre 14 est située au-dessus de la surface d'équilibre du bain 2. Le vase 10 comporte une autre ouverture raccordée à un tuyau 15 muni d'une vanne 16 dont l'ouverture et la fermeture sont effectuées à l'aide d'un organe de commande 17. L'extrémité libre du tuyau 15 est située au-dessus d'un récipient 18. Un appareil de dosage 19, dont la sortie électrique est reliée à l'organe 17, est muni d'une sonde 20 pénétrant à l'intérieur du vase 10. Un capteur 21 du niveau du bain 2 dans la cuve 1 est relié à un organe 22 commandant l'ouverture et la fermeture d'une vanne 23. Cette vanne est placée en série dans une canalisation 24 dont une extrémité est située au-dessus de la surface d'équilibre du bain 2 et dont l'autre extrémité est reliée à un réservoir non représenté. Un agitateur à hélice 25 entraîné par un moteur 26 est plongé dans le bain 2. Enfin, le dispositif comporte deux capteurs 27 et 28 mesurant respectivement la température et l'acidité (pH) du bain 2.

Le dispositif décrit ci-dessus et illustré par la figure fonctionne de la manière suivante.

On met en marche le four 4 de façon à porter la température du bain, mesurée par le capteur 27, à une valeur convenable prédéterminée. On ajuste ensuite le pH du bain, mesuré par le capteur 28, à un taux prédéterminé, par addition dans le bain d'acide acétique ou de soude diluée par exemple.

Dès l'ouverture de la vanne 23, une suspension aqueuse d'une poudre de déchets organiques s'écoule dans le bain 2 par la canalisation 24. La fermeture de la vanne 23 est commandée automatiquement par l'organe 22 dès que le niveau du bain 2, mesuré par le capteur 21, atteint la valeur désirée par l'opérateur.

On met en marche le moteur 26 de l'agitateur de façon à rendre le bain plus homogène. Sous l'action de la première enzyme immobilisée sur les granulés d'alumine, les particules des déchets organiques introduits dans le bain se transforment en un mélange de sucres qui entre en solution dans le bain pour former un sirop. Ce mélange contient en particulier un sucre fermentescible, apte à être transformé en alcool ou en méthane par fermentation.

On met en marche la pompe 12: les sucres en solution dans le bain 2 traversent les pores du support et de la membrane 8 du tube 5 vers le volume interne du tube dans le sens des flèches 29, alors que les granulés d'alumine γ et les particules des déchets organiques non transformés en sucre sont retenues dans le bain par les pores du support 7 du tube 5.

Le liquide aspiré dans le tube 5 arrive dans le vase d'expansion 10, puis est renvoyé dans la cuve 1 à travers le circuit de recyclage constitué par la canalisation 11, la pompe 12 et la canalisation 13.

Au cours de la traversée de la membrane 8 du tube, les sucres sont mis en contact avec la deuxième enzyme immobilisée dans cette membrane. Il en résulte un enrichissement du sirop en sucre fermentescible.

L'appareil de dosage 19 mesure le taux de sucre fermentescible dans le liquide présent dans le vase 10. Lorsque ce taux est supérieur à une valeur prédéterminée, l'organe 17 relié à la sortie de l'appareil 19 commande l'ouverture de la vanne 16. Le sirop enrichi en sucre fermentescible est recueilli dans le récipient 18.

Lorsque le niveau du bain 2 descend en dessous d'une valeur fixée par la sonde 21, l'organe 22 provoque de nouveau l'ouverture de la vanne 23.

Dans un autre mode de réalisation, la deuxième enzyme est immobilisée, comme la première enzyme, dans les granulés de céramique microporeux 3. Dans ce cas, le tube 5 ne comporte pas de membrane microporeuse 8. Le tube 5 sert uniquement à retenir les granulés et les déchets organiques subsistant dans le bain. La deuxième enzyme agit alors directement sur les sucres engendrés par l'action de la première enzyme dès la formation de ces sucres.

Pour immobiliser la première enzyme et éventuellement la deuxième enzyme dans les granulés d'alumine γ poreux, on met d'abord les enzymes en solution dans de l'eau, on règle le pH de la solution à une valeur convenable et on ajoute des produits stabilisants, par exemple des ions $Ca^{++}$ ou $Na^+$. Puis on verse les granulés dans la solution et on agite le bain pour augmenter l'homogénéité de la suspension: les enzymes sont alors adsorbées sur les granulés. Les enzymes non fixées peuvent être éliminées par lavage.

L'immobilisation de la deuxième enzyme dans la membrane du tube poreux s'effectue en préparant d'abord une solution aqueuse de la deuxième enzyme et en réglant le pH à une valeur convenable. On fait ensuite passer plusieurs fois la solution à travers la paroi du tube à l'aide d'un dispositif de pompage analogue à celui représenté sur la figure: les enzymes sont alors immobilisées dans les pores de la membrane. On peut éliminer par lavage les enzymes non fixées.

Pour augmenter la résistance mécanique de la membrane du tube, on réalise celle-ci avec de l'alumine γ frittée. Dans ce cas, on traite avantageusement le tube, avant immobilisation de la deuxième enzyme, avec un acide tel que l'acide phosphorique $PO_3H$. Ce traitement crée dans la membrane des sites actifs superficiels qui favorisent l'immobilisation de la deuxième enzyme.

Après une certaine durée de fonctionnement, l'élimination des enzymes désactivées immobilisées dans les pores de la membrane du tube peut s'effectuer par exemple en chauffant le tube dans l'air à une température de l'ordre de 600 à 700°C.

En principe, étant donné le prix relativement faible des granulés d'alumine γ, il n'est pas rentable d'éliminer les enzymes désactivées en vue d'une nouvelle immobilisation d'enzymes. Cependant, cette élimination peut être éventuellement réalisée par un chauffage des granulés.

Le dispositif décrit ci-dessus présente les avantages suivants.

Ce dispositif est particulièrement simple, car il permet de réaliser, dans une même cuve, les deux réactions chimiques provoquées respectivement par la première et la deuxième enzyme. Il présente une excellente tenue mécanique et dimensionnelle. Comme il a été vu ci-dessus, il peut être facilement automatisé afin de rendre son fonctionnement continu.

La première enzyme est utilisée jusqu'à désactivation complète. La régénération des tubes poreux contenant des enzymes désactivées est simple et peu coûteuse.

Le rendement des réactions enzymatiques est augmenté du fait que les produits de réaction sont extraits dès leur formation. On sait en effet que les réactions enzymatiques sont inhibées par une élévation de la concentration en produits de réaction. Dans le dispositif décrit ci-dessus, les sucres formés par la réaction provoquée par la première enzyme sont immédiatement traités par la deuxième enzyme, ce traitement s'effectuant directement dans le bain ou par traversée de la membrane du tube; le sucre fermentescible résultant de la réaction provoquée par la deuxième enzyme est extrait du récipient 10 dès que la concentration en sucre fermentescible dépasse un taux prédéterminé.

Deux exemples d'utilisation du dispositif selon l'invention sont décrits ci-dessous.

*Exemple 1:*

Les déchets organiques à traiter sont constitués essentiellement par de l'amidon.

La première enzyme est alors l'$\alpha$-amylase qui transforme l'amidon en un mélange de maltose et de glucose. La réaction produite par l'$\alpha$-amylase a un rendement optimal pour une température comprise entre 65 et 80°C et un pH de 5,5 à 7, en présence d'ions $Ca^{++}$ et $Na^+$.

La deuxième enzyme est l'amyloglucosidase qui transforme le maltose en glucose. La réaction a un rendement optimal pour une température comprise entre 50 et 65°C et un pH compris entre 4 et 5.

Le bain 2 contenu dans la cuve 1 est maintenu, dans ce cas, à une température comprise entre 60 et 65°C, son pH étant compris entre 5 et 6.

Bien qu'un tel dispositif permette d'obtenir des solutions de glucose à un taux de 35 g/l, on se limite à une concentration de 15 g/l. En effet, l'activité des enzymes commence à baisser sensiblement au-delà de ce taux.

Un dispositif de ce type peut fonctionner pendant un mois sans addition d'enzymes avec un taux de transformation de 1 g d'amidon par heure et par gramme de granulés d'alumine $\gamma$.

*Exemple 2:*

Les déchets organiques à traiter sont constitués essentiellement par de la cellulose.

La première enzyme est la cellulase qui transforme la cellulose en un mélange de cellobiose et de glucose. La réaction a un rendement optimal pour une température comprise entre 40 et 50°C et un pH compris entre 4 et 5.

La deuxième enzyme est la $\beta$-glucosidase qui transforme la cellobiose en glucose. La réaction a un rendement optimal pour une température comprise entre 35 et 40°C et un pH compris entre 4 et 5.

On règle alors la température du bain à 40°C et son pH entre 4 et 5.

Un tel dispositif permet d'obtenir une solution de glucose à un taux de 7 g/l. Il peut fonctionner pendant 10 d avec un taux de transformation de 10 mg de cellulose par heure et par gramme de granulés d'alumine $\gamma$. On peut augmenter le rendement en effectuant un traitement préalable de la cellulose par un acide dilué.

Le dispositif selon l'invention peut être utilisé aussi pour le traitement des déchets à base d'hémicellulose.

Le dispositif selon l'invention peut donc être appliqué au traitement de nombreux types de déchets agricoles, forestiers ou aquatiques, afin d'obtenir une solution sucrée qui peut être transformée par fermentation en alcool ou en méthane.

## Revendications

1. Dispositif pour obtenir du sucre fermentescible à partir de déchets organiques, comportant:
— des moyens pour mettre en contact un liquide contenant en suspension des déchets organiques avec une première enzyme apte à transformer ces déchets en un mélange de sucres, ce mélange contenant un sucre fermentescible, et
— des moyens pour mettre en contact le mélange avec une deuxième enzyme immobilisée dans une céramique poreuse, de façon à enrichir le mélange en sucre fermentescible,
caractérisé en ce qu'il comporte:
— une cuve (1) contenant un bain dudit liquide (2) dans lequel est immergée une céramique microporeuse, la deuxième enzyme étant immobilisée dans cette céramique,
— des granulés microporeux (3) de céramique en suspension dans le bain, la première enzyme étant immobilisée dans ces granulés,
— un tube (5) dont la paroi poreuse comporte un support tubulaire (7) en céramique macroporeuse, une cloison étanche (6) pour obturer hermétiquement une extrémité du tube, l'autre extrémité du tube étant reliée à une canalisation de recyclage (11, 13) munie d'une pompe (12) et aboutissant au-dessus du bain, le tube étant immergé dans le bain et la pompe étant capable d'aspirer dans la canalisation la partie liquide du bain à travers la paroi du tube et de renvoyer le liquide aspiré dans le bain,
— des moyens (23, 24) pour verser dans le bain (2) le liquide contenant les déchets organiques en suspension, les pores du support (7) du tube (5) étant capables de retenir, pendant l'aspiration, les granulés et les déchets organiques subsistant dans le bain,
— des moyens (27, 28) pour maintenir la température et l'acidité du bain contenant les granulés et les déchets organiques à des valeurs prédéterminées qui permettent, d'une part, la transformation des déchets organiques en un mélange de sucres sous l'action de la première enzyme et, d'autre part, l'enrichissement du mélange en sucre fermentescible sous l'action de la deuxième enzyme, et

— des moyens commandables (15 à 18) pour prélever le sucre fermentescible à la sortie du tube.

2. Dispositif selon la revendication 1, caractérisé en ce que la deuxième enzyme est également immobilisée dans les granulés (3).

3. Dispositif selon la revendication 1, caractérisé en ce que la paroi du tube (5) comporte une membrane (8) tubulaire intérieure en céramique microporeuse, cette membrane étant fixée sur la surface interne du support macroporeux (7), la deuxième enzyme étant immobilisée dans cette membrane.

4. Dispositif selon la revendication 3, caractérisé en ce que la membrane (8) du tube est en alumine γ.

5. Dispositif selon la revendication 1, caractérisé en ce que les granulés (3) sont en alumine γ.

6. Dispositif selon la revendication 1, caractérisé en ce qu'il comporte en outre un appareil (19) pour mesurer le taux de sucre fermentescible à la sortie du tube (5), lesdits moyens pour recueillir le sucre fermentescible étant commandés lorsque ce taux atteint une valeur donnée.

7. Dispositif selon la revendication 6, caractérisé en ce qu'il comporte un vase d'expansion (10) disposé en série dans la canalisation (11) entre le tube (5) et la pompe (12), ledit appareil (19) étant disposé de façon à mesurer le taux de sucre fermentescible dans le vase (10), lesdits moyens pour prélever le sucre fermentescible comportant une vanne (16) commandable disposée sur un canal de sortie (15) du vase d'expansion.

8. Dispositif selon la revendication 1, caractérisé en ce qu'il comporte un agitateur (25) disposé dans le bain (2) pour maintenir les granulés et les déchets organiques en suspension homogène dans le bain.

9. Dispositif selon la revendication 4, caractérisé en ce que la membrane (8) du tube (5) est réalisée en alumine γ frittée, cette membrane comportant des sites actifs superficiels aptes à favoriser l'immobilisation de la deuxième enzyme.

## Patentansprüche

1. Vorrichtung zur Herstellung von gärungsfähigem Zucker aus organischen Abfällen, mit

— Mitteln, um eine organische Abfälle in Suspension enthaltende Flüssigkeit mit einem ersten Enzym in Kontakt zu bringen, das diese Abfälle in eine Zuckermischung umwandeln kann, wobei diese Mischung einen gärungsfähigen Zucker enthält, und

— Mitteln, um die Mischung mit einem zweiten Enzym in Kontakt zu bringen, das in einer porösen Keramiksubstanz fixiert ist, um die Mischung mit gärungsfähigem Zucker anzureichern,

dadurch gekennzeichnet, dass sie aufweist:

— einen Behälter (1), der ein Bad der genannten Flüssigkeit (2) aufweist, in das eine mikroporöse Keramiksubstanz eingetaucht ist, wobei das zweite Enzym in dieser Keramiksubstanz fixiert ist,

— mikroporöse Keramikkörnchen (3) in Suspension in dem Bad, wobei das erste Enzym in diesen Körnchen fixiert ist,

— ein Rohr (5), dessen poröse Wand einen rohrförmigen Träger (7) aus makroporöser Keramiksubstanz aufweist, eine dichte Trennwand (6), um ein Ende des Rohrs hermetisch zu verschliessen, während das andere Ende des Rohrs mit einer Recycling-Leitung (11, 13) verbunden ist, die mit einer Pumpe (12) versehen ist und oberhalb des Bads mündet, wobei das Rohr in das Bad eingetaucht ist und die Pumpe in die Leitung den flüssigen Teil des Bads durch die Wand des Rohrs ansaugen und die angesaugte Flüssigkeit wieder in das Bad zurückschicken kann,

— Mittel (23, 24), um in das Bad (2) die die organischen Abfälle in Suspension enthaltende Flüssigkeit zu giessen, wobei die Poren des Trägers (7) des Rohrs (5) während des Ansaugens die in dem Bad verbliebenen Körnchen und die organischen Abfälle zurückhalten können,

— Mittel (27, 28), um die Temperatur und den Säuregrad des die Körnchen und die organischen Abfälle enthaltenden Bads auf solchen vorbestimmten Werten zu halten, die einerseits die Umwandlung der organischen Abfälle in eine Mischung von Zuckern unter Einwirkung des ersten Enzyms und andererseits die Anreicherung der Mischung mit gärungsfähigem Zucker unter Einwirkung des zweiten Enzyms ermöglichen, und

— steuerbare Mittel (15 bis 18), um den gärungsfähigen Zucker am Ausgang des Rohrs zu entnehmen.

2. Vorrichtung nach Anspruch 1, dadurch gekennzeichnet, dass das zweite Enzym ebenfalls in den Körnchen (3) fixiert ist.

3. Vorrichtung nach Anspruch 1, dadurch gekennzeichnet, dass die Wand des Rohrs (5) eine rohrförmige innere Membran (8) aus mikroporösem Keramikmaterial aufweist, die an der inneren Oberfläche des makroporösen Trägers (7) befestigt ist, wobei das zweite Enzym in dieser Membran fixiert ist.

4. Vorrichtung nach Anspruch 3, dadurch gekennzeichnet, dass die Membran (8) des Rohrs aus γ-Aluminiumoxid ist.

5. Vorrichtung nach Anspruch 1, dadurch gekennzeichnet, dass die Körnchen (3) aus γ-Aluminiumoxid sind.

6. Vorrichtung nach Anspruch 1, dadurch gekennzeichnet, dass sie ausserdem einen Apparat (19) aufweist, um den Gehalt an gärungsfähigem Zucker am Ausgang des Rohrs (5) zu messen, wobei die Mittel zur Entnahme des gärungsfähigen Zuckers dann angesteuert werden, wenn dieser Gehalt einen bestimmten Wert erreicht.

7. Vorrichtung nach Anspruch 6, dadurch gekennzeichnet, dass sie ein Ausdehnungsgefäss (10) aufweist, das in Reihe in der Leitung (11) zwischen dem Rohr (5) und der Pumpe (12) angeordnet ist, wobei der Apparat (19) so angeordnet ist, dass er den Gehalt an gärungsfähigem Zukker in dem Gefäss (10) misst, und wobei die Mittel zur Entnahme des gärungsfähigen Zuckers ein steuerbares Ventil (16) aufweisen, das auf einem Ausgangskanal (15) des Ausdehnungsgefässes angeordnet ist.

8. Vorrichtung nach Anspruch 1, dadurch gekennzeichnet, dass sie einen Rührer (25) aufweist, der in dem Bad (2) angeordnet ist, um die Körnchen und die organischen Abfälle in dem Bad in einer homogenen Suspension zu halten.

9. Vorrichtung nach Anspruch 4, dadurch gekennzeichnet, dass die Membran (8) des Rohrs aus gesintertem γ-Aluminiumoxid besteht, wobei diese Membran aktive oberflächliche Bereiche aufweist, die die Fixierung des zweiten Enzyms begünstigen.

## Claims

1. A device for obtaining fermentable sugar from organic wastes, including:
— means for putting a liquid, which contains organic wastes in suspension, into contact with a first enzyme which is capable of transforming said wastes into a mixture of sugars, which mixture contains a fermentable sugar, and
— means for putting said mixture into contact with a second enzyme trapped in a porous ceramic substance, so as to thereby enrich the mixture with fermentable sugar,
characterized in that it includes:
— a tank (1) containing a liquid bath of said liquid (2) in which a microporous ceramic substance is immersed, the second enzyme being trapped in said ceramic substance,
— microporous grains (3) of ceramic substance in suspension in the bath, the first enzyme being trapped in these grains,
— a tube (5), the porous wall of which includes a tubular support (7) made of macroporous ceramic substance, a sealed partition (6) for hermetically closing one end of the tube, the other end of the tube being connected to a recycling pipe (11, 13) equipped with a pump (12) and ending above the bath, the tube being immersed in the bath and the pump being capable of sucking the liquid part of the bath into the pipe through the wall of the tube and to return the sucked-up liquid into the bath,
— means (23, 24) for pouring the liquid containing the organic wastes in suspension, into the bath (2), the pores of the support (7) of the tube (5) being capable of retaining, during suction, the grains and the organic wastes which still remain in the bath,
— means (27, 28) for maintaining the temperature and the acidity of the bath which contains the grains and the organic wastes at predetermined values which allow on the one hand the transformation of the organic wastes into a mixture of sugars under the action of the first enzyme, and on the other hand the enrichment of the mixture with fermentable sugar under the action of the second enzyme, and
— controllable means (15 to 18) for drawing off the fermentable sugar at the outlet of said tube.

2. A device according to Claim 1, characterized in that the second enzyme is also trapped in the grains (3).

3. A device according to Claim 1, characterized in that the wall of the tube (5) has a tubular inner membrane (8) made of a microporous ceramic substance, said membrane being fixed on the inner surface of the macroporous support (7), the second enzyme being trapped in said membrane.

4. A device according to Claim 3, characterized in that the membrane (8) of the tube is made of γ-alumina.

5. A device according to Claim 1, characterized in that the grains (3) are γ-alumina grains.

6. A device according to Claim 1, characterized in that it further includes an apparatus (19) for measuring the concentration of fermentable sugar at the outlet of the tube (5), said means for drawing off the fermentable sugar being controlled when said concentration reaches a given value.

7. A device according to Claim 6, characterized in that it includes an expansion chamber (10) disposed in series in the pipe (11) between the tube (15) and the pump (12), said apparatus (19) being disposed so as to measure the concentration of fermentable sugar in the expansion chamber (10), said means for drawing off the fermentable sugar including a controllable valve (16) disposed on an outlet pipe (15) of the expansion chamber.

8. A device according to Claim 1, characterized in that it includes a stirring unit (25) disposed in the bath (2) to keep the grains and the organic wastes in a homogenous suspension in the bath.

9. A device according to Claim 4, characterized in that the membrane (8) of the tube (5) is made of sintered γ-alumina, said membrane having active surface sites which are capable of promoting trapping of the second enzyme.